# EUROPÄISCHE PATENTSCHRIFT

(11) **EP 2 276 752 B1**
(45) Veröffentlichungstag und Bekanntmachung des Hinweises auf die Patenterteilung: **26.07.2017**
(21) Anmeldenummer: 09731220.1
(22) Anmeldetag: 09.04.2009
(51) Int. Cl.: A61K 51/10, C07D 257/10, C07D 257/02, A61K 51/04

(54) **VERBINDUNG AUS EINEM METALLION UND EINEM MARKIERUNGSVORLÄUFER MIT ZWEI ODER MEHREREN TARGETINGVEKTOREN UND VERWENDUNG DER VERBINDUNG**
COMPOUND COMPOSED OF A METAL ION AND A MARKER PRECURSOR HAVING TWO OR MORE TARGETING VECTORS, AND USE OF THE COMPOUND
COMPOSÉ CONSTITUÉ D'UN ION MÉTALLIQUE ET D'UN PRÉCURSEUR DE MARQUAGE, COMPORTANT DEUX OU PLUSIEURS VECTEURS CIBLES, ET UTILISATION DU COMPOSÉ

(30) Priorität: 11.04.2008 DE 102008018634; 04.09.2008 DE 102008045937
(43) Veröffentlichungstag der Anmeldung: 26.01.2011
(73) Patentinhaber: Johannes Gutenberg-Universität Mainz, 55128 Mainz (DE)
(72) Erfinder: RÖSCH, Frank, 55270 Zornheim (DE); RISS, Patrick, Cambridge CB1 9 LD (GB); BURCHARDT, Carsten, 91052 Erlangen (DE)
(74) Vertreter: Plate, Jürgen
(86) Internationale Anmeldenummer: PCT/EP2009/002636
(87) Internationale Veröffentlichungsnummer: WO 2009/124764

(56) Entgegenhaltungen:
- WO-A-93/06868
- WO-A-2004/021996
- DE-A1- 19 729 013

## Beschreibung

Die Erfindung betrifft eine Verbindung aus einem Metallion und einem Markierungsvorläufer mit zwei oder mehreren voneinander verschiedenen Targetingvektoren und die Verwendung der Verbindung.

In der medizinischen Bildgebung werden Metallkomplexe zur Funktions- und Lokalisationsdiagnostik, sowie in der Therapie angewandt.

In der Diagnostik stellen die bildgebenden Verfahren Positron-Emission-Tomography (PET) und Single-Photon-Emission-Computed-Tomography (SPECT) nach dem Tracer-Prinzip vor allem die biochemische Funktion eines Organs oder Gewebes dar, während die bildgebenden Verfahren Computertomographie (CT) und Kernspintomographie (MRI) die morphologische Struktur beispielsweise von Organen oder des Knochenskeletts zeigen. Wird beispielsweise ein Radiopharmakon verwendet, das bevorzugt von knochenbildenden Zellen aufgenommen wird, so zeigt normales Knochengewebe in der Tomographie niedrige Aktivität. Im Gegensatz zu krankhaftem Knochengewebe, dessen erhöhte Aktivität verstärkten Knochenumbau anzeigt, beispielsweise verursacht durch Krebs, gutartigen Knochentumor, Frakturen, Arthrosen oder Entzündungen.

### Aminosäuren zur Tumordiagnostik

Aminosäuren stellen, vor allem in der Onkologie, nützliche Targetingvektoren für PET- bzw. SPECT dar. Sie werden über verschiedene Aminosäuretransportsysteme in Zellen transportiert und dort in bestimmten Fällen in der Proteinbiosynthese weiter zu Proteinen umgesetzt. Das schnelle Wachstum dieser Tumore bedingt neben einer erhöhten Proteinsyntheserate auch eine erhöhte Aminosäuretransporteraktivität und -dichte. Die erhöhte Anreicherung der radioaktiv markierten Aminosäure in Tumorzellen begründet sich also in der Regel in der hohen Proliferationsrate maligner Tumore. Die Akkumulation erfolgt im Idealfall durch den Einbau der markierten Aminosäuren in Proteine. Parallel dazu ist es möglich, dass markierte Aminosäuren in die Zelle transportiert, dort aber nicht mehr in Proteine eingebaut werden. Studien zeigen einen direkten Zusammenhang zwischen Proteinsyntheserate und Aminosäuretransporteraktivität. Daher ist es für eine Anreicherung der markierten Aminosäure in der Zelle nicht unbedingt notwendig, dass diese auch in Proteine eingebaut wird, was eine Variation der Struktur der radioaktiv markierten Aminosäure in jedem Maß zulässt, das durch den Aminosäuretransporter toleriert wird. Dies ist beispielsweise für das O-(2-[¹⁸F]Fluorethyl)-L-Tyrosin (FET) der Fall.

Momentan werden für die PET bereits ¹¹C- oder ¹⁸F- markierte Aminosäuren, wie das L-[Methyl-¹¹C]Methionin (MET), das 2-[¹⁸F]Fluor-L-Tyrosin, das O-(2-[¹⁸F]Fluorethyl)-L-Tyrosin (FET) und andere ¹¹C- bzw. ¹⁸F-markierte Aminosäuren als Tumortracer klinisch eingesetzt. Man geht davon aus, dass die Bindung eines Tracers an einen Aminosäuretransporter ausreicht, um eine Anreicherung im Vergleich zum normalen Gewebe zu erhalten und den Tumor abbilden zu können. Dies gewährt einen noch deutlich größeren Spielraum bei der Variation der eingesetzten Strukturen.

Zusätzlich wurden radioaktiv markierte künstlichen Aminosäuren beschrieben, wie beispielsweise 1-Amino-3-[¹⁸F]fluorcyclobutan-1-carbonsäure (FACBC), die über den Aminosäuretransporter in die Zelle transportiert werden und sich dort akkumulieren.

Eine besondere Bedeutung besitzen radioaktiv markierte Aminosäuren bei der Diagnostik von Hirntumoren, da ein gesundes Hirn einen niedrigen Stoffwechsel an Aminosäuren besitzt und dort lokalisierte maligne Tumore einen deutlich höheren Aminosäurestoffwechsel aufweisen.

Klinisch etabliert ist beispielsweise der Zusammenhang von PET-Tumordiagnostik mit markierten Aminosäuren als prä-therapeutische Diagnostik zur Vorbereitung chirurgischer oder strahlen-basierter Therapien.

Dies erfordert eine hohe Verfügbarkeit der markierten Aminosäuren in der klinischen Routine.

### Metall-basierte Radiopharmaka

In der funktionellen Bildgebung mittels PET oder SPE(C)T werden beispielsweise Biomoleküle bekannter Funktion (Nukleotide, Neurotransmitter oder Zucker) radioaktiv markiert, um intelligente Sonden zu erhalten, die in ihrer Funktion mit dem Original identisch (Isotopentracer) oder ihm ausreichend ähnlich sind (Analogtracer). In vielen Fällen besitzen Analogtracer spezifische biologische Funktionen, die einen wertvollen Rückschluss auf das Verhalten der Originale erlauben.

Die Einführung eines sperrigen Metallkomplexes in ein biologisch relevantes (kleines) Molekül ist jedoch oft mit drastischen Änderungen der Moleküleigenschaften verbunden, da sich physikalische Parameter (Lipophilie, Solvatisierung, molare Masse, Diffusions-und Permeationseigenschaften u.Ä.) wie auch chemisch-physikalische Parameter (Aktivierungsenergien von essentiellen Konformationsänderungen, Elektronenverteilungen, Komplexstabilitäten und -bildungsgeschwindig-keiten o.a.) signifikant ändern.

Metall-Komplexbildner besitzen andererseits eine wichtige Funktion in der diagnostischen funktionellen Bildgebung. Ihre Metallkomplexe können als Kontrastmittel für die Computertomographie (Röntgentomographie), für die Kernspintomographie (MRI) und die Bildgebung mittels Ultraschall eingesetzt werden, sowie als Komplexe radioaktiver Metalle in der nuklearmedizinischen Diagnostik und Therapie. Komplexe mit stabilen Lanthaniden oder anderen schweren Elementen mit Atomgewichten größer als das von Iod (M_{Atom}> lod) besitzen eine hohe Absorption für Röntgenstrahlung und können somit als Röntgenkontrastmittel dienen. Komplexe mit hoher Röntgenabsorption besitzen zum Teil auch ausreichende Ultraschall-Streu- oder Reflektionseigenschaften, um als Ultraschallkontrastmittel eingesetzt werden zu können. Paramagnetische Elemente mit symmetrischem elektronischem Grundzustand (z.B. Gd(III) oder Fe(III)) bilden Komplexe mit Chelatoren, die als Spin-Relaxations-Katalysatoren für die Anwendung in der MRI eingesetzt werden können. Durch die Kombination von Chelatoren mit paramagnetischen Elementen, die einen unsymmetrischen Elektronengrundzustand besitzen, werden shift-Reagenzien für die MRI erhalten. Zahlreiche Lanthanidkomplexe makrozyklischer Chelatoren besitzen vorteilhafte (z.B. pH-abhängige) Absorptions- und Fluoreszenzeigenschaften im Bereich des sichtbaren Lichts. Sie eignen sich insbesondere für die Anwendung in modernen, lichtmikroskopischen Methoden der molekularen Bildgebung.

Voranstehend genannte Chelatoren und Komplexe können auch bifunktionell sein, d.h. neben der Komplexierung des Metallions können sie eine weitere Funktionalität besitzen, die neue chemische, physikalische oder biologische Eigenschaften mit sich bringt. Die Komplexbildner in der vorliegenden Erfindung sollen zwei solcher Funktionalitäten beinhalten, die als sogenannte Targetingvektoren (TV), die selektive Anreicherung des Metallkomplexes in einem definierten Gewebe *in vivo* bewirken. Dadurch erhält der Komplex für die funktionelle Bildgebung entscheidende Vorteile.

### ⁶⁸Ga als Radionuklid-basierter und leicht verfügbarer Positronen-Emitter für die Synthese von PET-Radiopharmaka

In der nuklearmedizinischen Diagnostik bzw. Therapie können Gamma- oder Positronenemitter für die Bildgebung mittels SPE(C)T (z.B. mit ^{99m}Tc oder ¹¹In) und Positronen-Emissions-Tomographie (PET) (z.B. mit ⁶⁸Ga und ⁶⁴Cu) eingesetzt werden. Dagegen besitzen α-(¹⁷⁷Lu) oder ß-Emitter (⁹⁰Y) zum Teil vorteilhafte Eigenschaften für die Endoradiotherapie (ERT).

Das Positronen-emittierende ⁶⁸Ga mit einer physikalischen Halbwertszeit T_{1/2} = 67,7 min ist von großer praktischer Bedeutung für die klinische Diagnostik mittels PET.

Den Radionuklidgeneratoren zur Gewinnung von radioaktiven metallischen Radionukliden liegt ein Konzept der effektiven radiochemischen Separation von Mutter- und Tochter-Radionuklidsystemen in solcher Weise zugrunde, dass das Tochternuklid in chemisch und radiochemisch möglichst reiner Form erhalten werden soll.

Im Vergleich zu in-house-Radionuklidproduktionsanlagen wie Beschleunigern oder Nuklearreaktoren bietet die Verfügbarkeit kurzlebiger Radionuklide von Radionuklidgeneratoren eine preiswerte und einfache Alternative. Die Entwicklung von Radionuklidgeneratoren war und ist stets geprägt durch das wachsende Spektrum der Anwendung von Radionukliden und markierten Pharmaka in der Medizin, insbesondere für die nuklearmedizinische Diagnostik und Therapie. Diese wachsende Verfügbarkeit von Radionuklidgeneratoren wiederum hat eine breite Entwicklung von neuen biologisch relevanten Molekülen stimuliert, die mit den Radionukliden aus Radionuklidgeneratoren markiert werden.

Die breite Nutzung des ⁹⁹Mo/^{99m}Tc-Generatorsystems in der Nuklearmedizin ist ein typisches Beispiel für die Anwendung von Radionuklidgeneratoren in Klinika und bei Herstellern einer großen Palette diagnostischer Radiopharmaka. Mehr als 35 000 diagnostische Studien mit ^{99m}Tc werden täglich allein in den USA, das sind mehr als 12 Millionen Anwendungen pro Jahr, geschätzter Weise durchgeführt.

Radionuklidgenerator-Entwicklungen sind oft systematisiert worden. Detaillierte Berichte hierüber haben sich verschiedenen Aspekten gewidmet: Mutter-Tochter Halbwertszeiten, Reaktor-produzierte Nuklide, Beschleuniger-produzierte Nuklide, Zyklotron-Produktion von Generatormutter-Nukliden, ultrakurzlebige Generator-produzierte Radionuklide, Generator-basierte Positron-emittierende Radionuklide und klinische Anwendungen.

Inzwischen wurden verschiedene andere Generatorsysteme entwickelt und einige davon haben signifikante praktische Bedeutung erlangt. Zurzeit bieten ⁶⁸Ge (T_{1/2} = 270.95 d) / ⁶⁸Ga (T_{1/2} = 67,7 min)-Generatorsysteme eine neue, vielversprechende Perspektive zur qualitativ und quantitativ verbesserten Verfügbarkeit neuer PET-Radiopharmaka.

### ⁶⁸Ga-basierte Tumordiagnostika

Seit einigen Jahren steht mit ⁶⁸Ga ein Generatornuklid auch für die PET zur Verfügung. Erste Generatorsysteme sind kommerziell erhältlich. Die Routineproduktion eines tumoraffinen Peptides zur bildgebenden Diagnostik vor allem neuroendokriner Tumore ist in Form des [⁶⁸Ga]DOTATOC (⁶⁸Ga-DOTA-DPhe¹-Tyr³-octreotid) grundsätzlich etabliert.

Im Fall der Synthese bzw. Anwendung von Ga-Radiopharmaka kann zwischen einfachen Ga-Ligand-Komplexen (z.B.[^{67,68}Ga]Citrat) und markierten Targetingvektoren (z.B. markierten Peptide wie [^{67,68}Ga]DOTATOC) unterschieden werden. Vor allem die zweite Kategorie bietet die interessantesten und vielfältigsten Möglichkeiten zur nuklearmedizinischen Diagnostik. Chemisch liegt diesem Ansatz die Verwendung bifunktioneller Chelatoren zugrunde.

Geeignete bifunktionelle Chelatoren müssen in der Lage sein, das ⁶⁸Ga mindestens über den Zeitraum einer PET-Untersuchung stabil zu komplexieren und gleichzeitig die Möglichkeit besitzen, kovalent an die benötigten Targetingvektoren gebunden zu werden. Hier hat sich für viele Fragestellungen 1,4,7,10-Tetraazacyclododekan-1,4,7,10-tetraessigsäure (DOTA), ein makrozyklischer Chelatligand, der bereits in der Komplexierung hochkoordinierender Lanthanide Verwendung findet und präparativ leicht zugänglich ist, bewährt.

Da der Chelator acht Koordinationsstellen besitzt und das für die Markierung zugängliche Ga(III) sechsfach koordiniert, verbleiben zwei der acht Koordinationsstellen im DOTA frei. Bei diesen handelt es sich um zwei Carboxylgruppen der Essigsäurereste, welche beispielsweise für die Konjugation mit einem Biomolekül zur Verfügung stehen. Im Falle des [⁶⁸Ga]DOTATOC wird beispielsweise nur eine der zwei freien Carboxylgruppen verwendet, um den TV (DPhe¹-Tyr³-Octreotid) an den Galliumkomplex zu binden. Die verbleibende freie Carboxylgruppe liegt bei physiologischem pH als Carboxylat vor, wodurch sich die chemischen Eigenschaften gegenüber vollständig koordinierten Verbindungen deutlich verändern. Dennoch weist [⁶⁸Ga]DOTATOC gegenüber seinem SPECT-Analogon C¹¹In]Octreoscan eine erheblich bessere Affinität auf, so dass nicht generell auf eine Verschlechterung der pharmakologischen Eigenschaften geschlossen werden kann.

Einige tripodale, tetraedrisch koordinierenden Chelatoren, insbesondere solche die Schwefeldonoren beinhalten, sind ebenfalls bekannt, werden aber aufgrund ihrer zumeist aufwendigen Synthese nicht eingesetzt.

Für die Kopplung von Ga-Targetingvektoren verwendet man zur Zeit in der Regel das kommerziell erhältliche DOTA und seine Derivate. Das neun-Ring-analoge NOTA gilt ebenfalls als sehr geeignet, konnte sich jedoch bisher aufgrund seiner aufwendigen synthetischen Darstellung noch nicht durchsetzen.

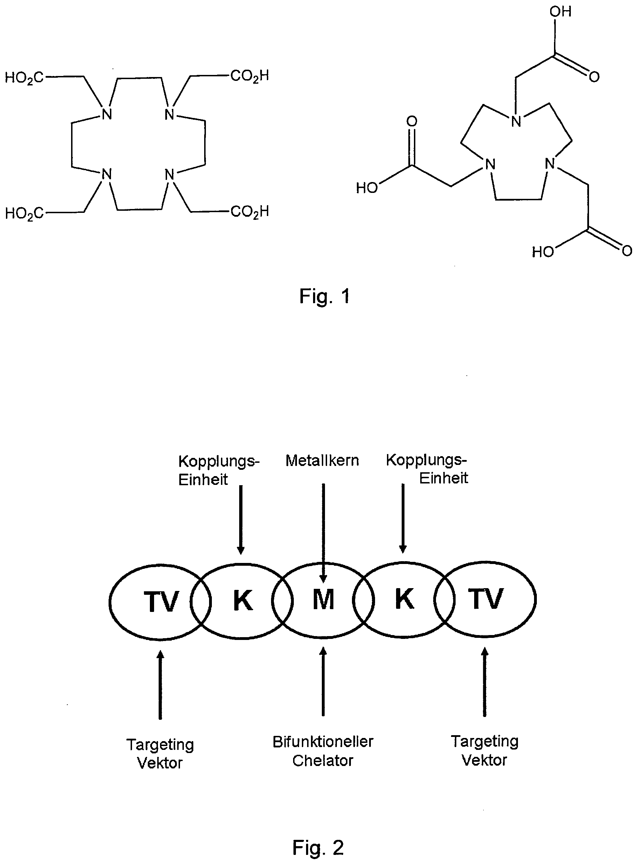

1,4,7,10-Tetraazacyclododekan-1,4,7,10-tetraessigsäure (DOTA) und das ebenfalls oft verwendete 1,4,7-Triazacyclononan-1,4,7-triessigsäure (NOTA) sind in Fig. 1 dargestellt.

Aufgabe der Erfindung ist die Schaffung von Verbindungen aus einem Metallion und einem Markierungsvorläufer bestehend aus dem bifunktionellen Chelator mit konjugierten Targetingvektoren.

Dabei soll es sich zum einen um Derivate handeln, bei denen zwei oder mehrere Aminosäure-Targetingvektoren pro Chelatormolekül eingesetzt werden. (Fig. 2)

Diese Aufgabe wird in der Weise gelöst, dass der Markierungsvorläufer aus einem bifunktionellen makrozyklischen Chelator, einer Kopplungseinheit und zumindest zwei verschiedenen Targetingvektoren aufgebaut ist.

Das Metallion ist insbesondere ein Radionuklid.

In Ausgestaltung der Erfindung umfasst der makrozyklische Chelator 9- bis 16-gliedrige Polyazamakrozyklen.

In vorteilhafter Weise ist der Chelator in der Verbindung ein makrozyklisches Polyamin, das an allen Stickstoffatomen substituiert ist, wobei das zentrale Metallion mit den benachbarten Stickstoffatomen 5- bzw. 6-Ring-Chelate bildet. Insbesondere ist bzw. sind an den Chelator eine bzw. mehrere Kombinationen aus Spacer, Linker und zwei oder mehrerer Aminosäuren als Targetingvektor gebunden.

Die Konjugation der beiden oder mehrerer Targetingvektoren erfolgt jeweils über verschiedene Ringstickstoff-Atome des makrozykischen Chelators und / oder verschiedener Kohlenstoffatome des Chelators.

In Ausgestaltung der Erfindung wird ein Chelator verwendet, der aus Polyaminopolycarbonsäuren und gegebenenfalls komplexbildenden Donoren aus der Gruppe CNHOH, CSOH, COOH, CH₂OH, CH₂SH, PO(OH)₂, CH₂SO₃H aufgebaut ist. Des Weiteren kann ein Chelator verwendet werden, der aus Polyaminopolycarbonsäuren aufgebaut ist, die zusätzlich oder anstelle einer oder mehreren Carbonsäureeinheiten auch SH-Gruppen enthalten.

Die erfindungsgemäßen Verbindungen weisen fünf-, sechs- oder mehrfachkoordinierende bifunktionelle Chelatoren sowie deren Komplexe mit Metallionen, insbesondere Radionukliden, zur Kopplung an Aminosäuren auf.

Die Chelatoren sind zurthermodynamisch stabilen und kinetisch inerten Komplexierung von Metallionen wie Radionukliden bzw. Radioisotopen, insbesondere von ⁶⁸Ga und anderen Radiogalliumisotopen befähigt und an die Chelatoren werden in geeigneter Weise Aminosäuren kovalent gebunden.

Die Chelatoren sind des Weiteren zur Bindung von mehr als einer Aminosäure je bifunktionellem Chelator geeignet. Dadurch wird dem resultierenden Komplex-Biomolekül-Konjugat eine molekulare Grundlage verliehen, die eine erhöhte Affinität zu einem Tumor bewirkt.

Die erfindungsgemäße Weiterbildung der Verbindung ergibt sich aus den Ansprüchen 11 bis 27.

Die Verbindung wird erfindungsgemäß zum Abbilden von Tumorgeweben mittels der Positronen-Emissions-Tomographie (PET) oder der Single-Photon-Emission-Computed-Tomography (SPECT), durch selektive Anreicherung des Metallkomplexes der Verbindung in dem zu untersuchenden Tumorgewebe in vivo verwendet.

In weiterer Verwendung zum Bestrahlen von Zielgewebe wird die Verbindung systemisch oder direkt in das Zielgewebe eingebracht. Insbesondere wird die Verbindung zum Abbilden von Herz-, Blutgefäß-, Hirn-, Nieren-, Lungen-, Leber- und Drüsengewebe sowie Knochen mittels PET verwendet.

Weitere Verwendungen der Verbindung sind in den Ansprüchen 31 bis 34 beschrieben.

Die erfindungsgemäßen Verbindungen eignen sich für die molekulare Bildgebung mit PET oder anderen Bildgebungsverfahren und werden in der nuklearmedizinischen Diagnostk und/oder Therapie eingesetzt. Insbesondere werden die Verbindungen zur nuklearmedizinischen Diagnostik von Tumoren mittels der Positronen-Emissions-Tomographie PET oder der Single-Photon-Emission-Computed-Tomography SPECT verwendet, die durch selektive Anreicherung in dem zu untersuchenden Zielgewebe *in vivo* ermöglicht wird.

Ebenso werden über die Verbindungen wie Radiogalliumpharmaka auf AminosäureBasis hinaus auch die zur Bildgebung bzw. Therapie von Tumoren relevanten Molekülen aus der Gruppe der Zucker, Purin- und Pyrimidinbasen, Nukleotide, Nukleoside und deren Derivate, Antikörper, Antikörperfragmente, Enzym- oder Rezeptorliganden als Targetingvektoren verwendet.

Darüber hinaus sind die Verbindungen in Gestalt von Radiogalliumpharmaka zur Markierung von Polymeren, Kolloiden, Nanopartikeln zur Darstellung bzw. Bildgebung von nicht-endogenen Strukturen verwendbar.

Des Weiteren werden die Verbindungen für die Endoradiotherapie (ERT) durch (in)-direktes Einbringen von Radiopharmaka in das Zielgewebe verwendet.

Nachfolgend werden die Bestandteile des Markierungsvorläufers näher beschrieben.

### a) Chelatoren

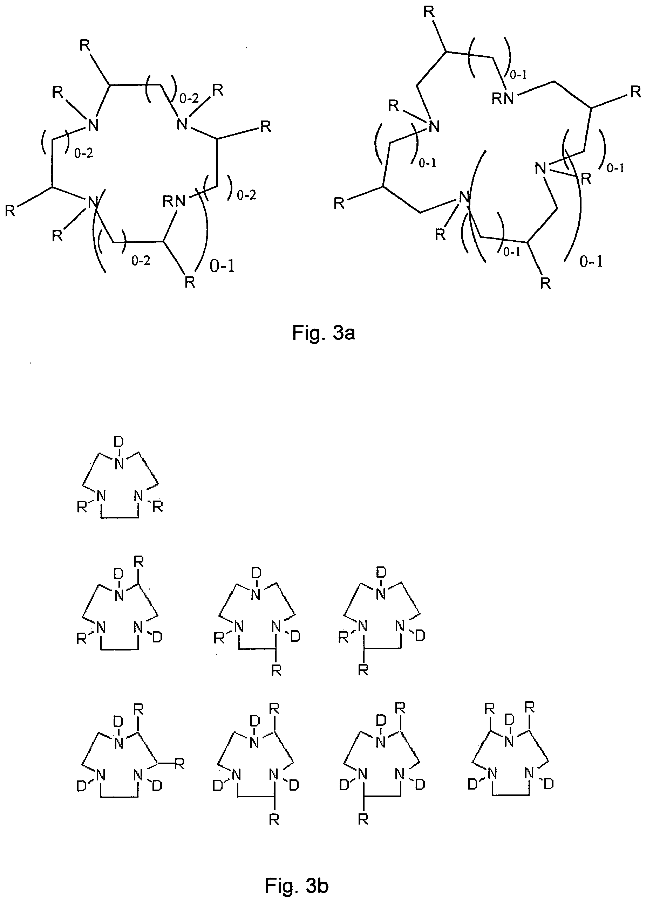

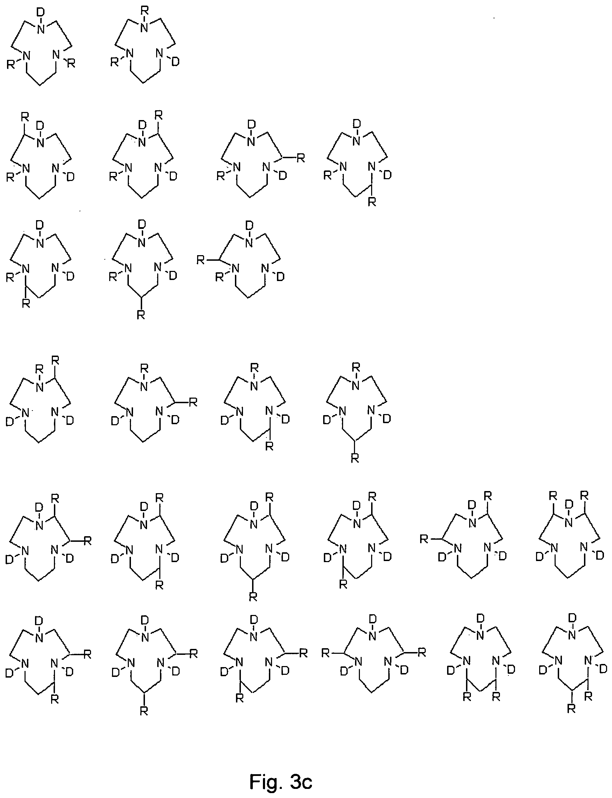

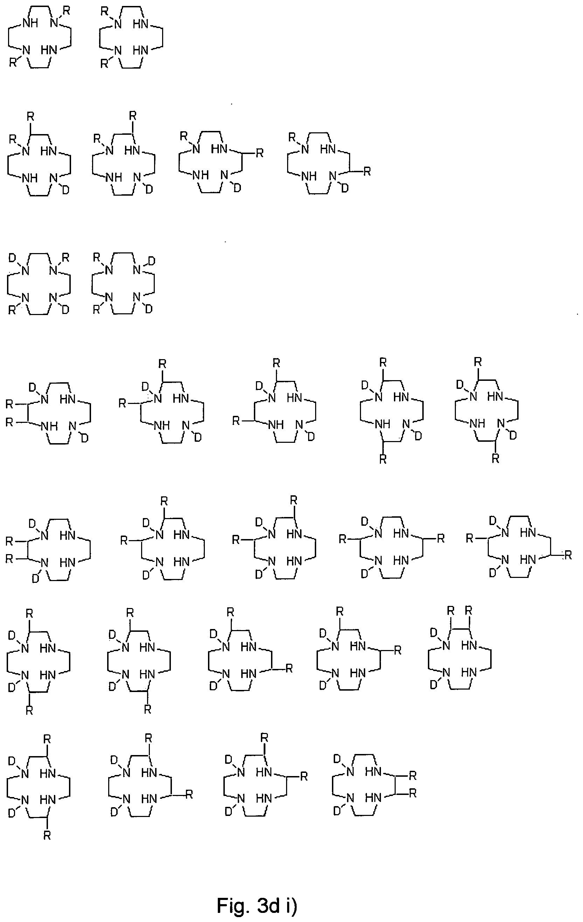

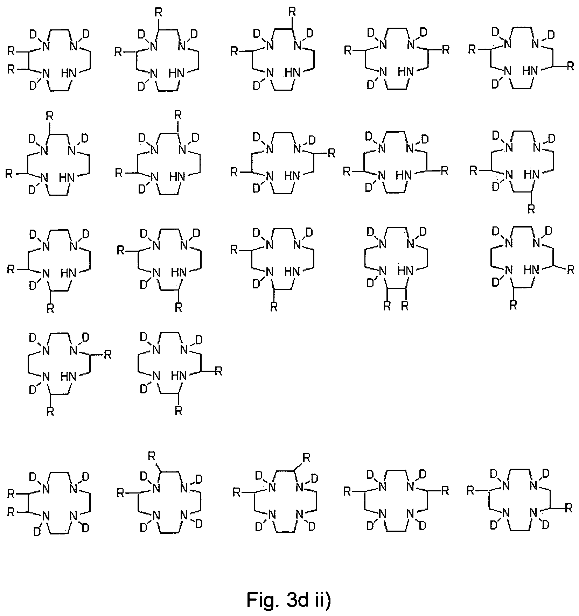

Allgemeine Strukturformeln der beschriebenen Chelatoren sind in Fig. 3a dargestellt. Der Chelator ist ein makrozyklisches Polyamin bestehend aus Polyaminopolycarbonsäuren und gegebenenfalls komplexbildenden Donoren, wie nachstehend erwähnt. Die Polyaminopolycarbonsäuren können zusätzlich oder anstelle einer oder mehrerer Carbonsäureeinheiten SH-Gruppen enthalten.

Die Reste R können in beiden Typen folgende Gruppen verkörpern: Wasserstoff, Alkyl, Aryl, Heteroaryl, Alkenyl, Alkinyl oder Kombination Kopplungseinheit-Aminosäure. Dabei können alle R-Gruppen identisch oder voneinander verschieden sein, solange das Produkt gleichzeitig mindestens zwei bioaffine Gruppen enthält und in der Lage ist, ein zentrales Metall bzw. Metallion im Chelator themodynamisch und kinetisch stabil zu komplexieren. Als Komplex-bildende Donorfunktionen kommen vor allem CNHOH, CSOH, COOH, CH₂OH, CH₂SH, PO(OH)₂, CH₂SO₃H oder vergleichbare Gruppen zum Einsatz.

### b) Kopplungen und Kopplungseinheiten

Verbindungen zwischen bifunktionellem Chelator und Targetingvektoren können die Kopplungseinheiten bestehend aus Spacer und / oder Linker enthalten.

Der Spacer ist aus linearen, verzweigten oder heterosubstituierten, gesättigten, ungesättigten oder aromatischen Kohlenwasserstoffen aller Oxidationsstufen oder aus Zuckern aufgebaut.

Der Spacer ist in der Regel eine lineare, verzweigte oder heterosubstituierte Alkyl-, Alkylen-, Alkyliden-, Aryl-, Polyether- oder Peptidkette, die kovalent unter Beibehaltung der Aminofunktionalität der Chelatorstickstoffe an zwei gegenüberliegende oder benachbarte Stickstoffatome im Makrozyklus des Chelators gebunden ist, um eine hinreichende Distanz, sowie ausreichende konformative Flexibilität zwischen dem bioaffinen Targetingvektor und dem körperfremden Komplex zu gewährleisten. Dabei ist die Spacerfunktion prinzipiell für die Einführung mindestens einer oder mehrerer Linkergruppen und damit von Targetingvektoren geeignet.

Geeignete Linker können eine Abgangsgruppe wie beispielsweise I, CI, Br, OTs, OMs, OTf, ONf oder ONs etc. oder eine aliphatische oder aromatische Amino-, Hydroxy-, Mercapto-, Alken-, Alkin-, CO₂H-, CONH₂-, CNOH-, Epoxy-, NCS-, COOH-, Aktivester-, NCO-, COX, B(OH)₂-, Maleinimid-, PR₃-, P(OR)₃-Gruppe, Isothiocyanat/Thioharnstoff oder Analoga enthalten, die eine Bindungsknüpfung mit einem Targetingvektor ermöglicht. Neben den beschriebenen Funktionen können die verbliebenen Reste R beispielsweise Wasserstoff, Alkyl-, Alkoxy-, Alkylthio-, Alkylamino-, Alkylformamido-, Aryl-, Aryloxy-Funktionen sein.

Unter "Abgangsgruppe" oder "Fluchtgruppe" ist der bei einer chemischen Reaktion abgespaltene Molekülbereich zu verstehen. Dabei kann es sich sowohl um einzelne Atome oder Ionen, aber vor allem um Moleküle und funktionelle Gruppen handeln.

Beispielhaft werden an 9-, 10- und 12-Ringen die verschiedenen Möglichkeiten zur Verbrückung zweier Targetingvektoren über den Chelator aufgezeigt. Analog dazu kann dieses Konzept auch auf 11-, 13-, 14-, 15- sowie 16-Ringe angewendet werden. Außerdem ist es möglich, nach diesem Konzept auch drei, vier oder mehr Targeting-Vektoren in das Molekül zu integrieren.

Die in den Fig. 3b-3d dargestellten Moleküle bestehen aus einem makrozyklischen Chelator sowie seinen Donorfunktionen D und Resten R, wobei, abweichend von Fig. 3a, R in den Fig. 3b-3d eine Kombination aus Kopplungseinheit und Targetingvektor darstellt. Optional kann R eine zusätzliche Donorfunktion beinhalten.
Die Donorfunktionen D können in jeder Struktur gleich oder voneinander verschieden sein; die Reste R können ebenfalls in jeder Struktur gleich oder voneinander verschieden sein. Dies gilt jeweils unter der Voraussetzung, dass jede Struktur mindestens 6 Donorfuntionen (inklusive der Ringstickstoffe) und mindestens 2 Targeting-Vektoren enthält.

### c) Aminosäuren

Als Targetingvektoren kommen primär Aminosäuren in Betracht. Diese werden über verschiedene Transportsysteme in die Zellen transportiert. In den nachfolgenden Ausführungsbeispielen wurden die proteinogenen Aminosäuren L-Tyrosin, L-Serin und L-Lysin, die für Aminosäuren mit aromatischer und aliphatischer Seitenkette stehen, verwendet. Der Transport in die Zellen erfolgt über den jeweiligen Aminosäuretransporter. Beim Tyrosin erfolgt der Transport größtenteils über das Transportsystem L für Aminosäuren mit voluminösen Seitenketten, beim Lysin überwiegend über den kationischen Aminosäuretransporter und beim Serin vornehmlich über das natriumabhängige Transportsystem A. Da diese Aufnahmemechanismen bekannt sind, kann in *in vitro*-Experimenten die spezifische Aufnahme der neuen Verbindungen bestätigt werden. Die Substraterkennung des Aminosäuretransporters geschieht über die α-Amino-Carbonsäure-Gruppe. Die Kopplung an den Spacer erfolgt in den Seitenketten der Aminosäuren.

Es sind prinzipiell alle Verbindung geeignet, die eine α-Amino-Carbonsäure-Gruppe beinhalten. Diese Aminosäuren können sowohl proteinogen als auch künstlich sein. Die proteinogenen Aminosäuren sind geeignet, solange sie über einen Spacer/-Linker an den Chelator gekoppelt werden können. Die Seitenketten können sowohl neutral als auch polar, aromatisch oder aliphatisch sein und Amino-, Carboxyl- und Hydroxylgruppen beinhalten. Außerdem kann die Aminosäure L-Prolin verwendet werden, die eine nichtessentielle, neutrale, genetisch codierte Aminosäure ist und als proteinbildende Aminosäure eine sekundäre Aminogruppe aufweist.

Geeignete künstliche Aminosäuren sind alle nicht proteinogenen α-Amino-Carbonsäuren, die eine Affinität zum Target besitzen.

Im Allgemeinen sind ein, zwei oder mehrere Aminosäuren als Targetingvektoren in dem Markierungsvorläufer vorhanden, wobei diese Aminosäure-Targetingvektoren identisch oder voneinander verschieden sind.

Der oder die Targetingvektoren besteht bzw. bestehen aus niedermolekularen Targetingvektoren aus der Gruppe anorganischer Komponenten wie beispielsweise Bisphosphonate, Zucker, DNA-Komponenten, Hypoxie-Tracern, extra- oder interzellulären Enzym- oder Rezeptorliganden, Zellmembran-Komponenten, Glutamin oder ähnlichen biologisch aktiven Strukturen gebildet sein.

Der Chelator ist mit beispielsweise zwei-, drei- oder vierwertigen metallischen Radionukliden markiert. Hierzu zählt u.a. dreiwertiges ⁶⁸Ga aus einem ⁶⁸Ge/Ga-Generatoreluat. Bevorzugt ist der Chelator mit einem Gallium-Radioisotop aus der Gruppe ⁶⁵Ga, ⁶⁶Ga, ⁶⁷Ga, ⁶⁸Ga, ⁷²Ga markiert.

Die Erfindung wird im folgenden anhand von Zeichnungen näher erläutert. Dabei zeigen:
- Fig. 1: 1,4,7,10-Tetraazacyclododekan-1,4,7,10-tetraessigsäure (DOTA) und 1,4,7-Triazacyclononan-1,4,7-triessigsäure (NOTA);
- Fig. 2: eine schematische Darstellung der Zielstruktur mit zwei, über den Chelator verbrückten Targetingvektoren;
- Fig. 3a: Allgemeine Strukturformeln der erfindungsgemäßen Chelatoren;
- Fig. 3b: Kopplungsmöglichkeiten von zwei Targetingvektor-beinhaltenden Resten R an 9-Ring-Chelatoren;
- Fig. 3c: Kopplungsmöglichkeiten von zwei Targeting-Vektor beinhaltenden Resten R an 10-Ring-Chelatoren;
- Fig. 3d: Kopplungsmöglichkeiten von zwei Targeting-Vektor beinhaltenden Resten R an 12-Ring-Chelatoren;
- Fig. 4: einen auf Cyclen basierenden Ansatz gemäß Beispiel 1, wobei die beiden Komplex-bildenden Carboxyldonoren sind an sich gegenüberliegenden Ringstickstoffatome gebunden. Als Spacer dient eine 3-Oxapentylkette. Zwei gleiche Aminosäuren werden als Targetingvektoren verwendet. Die nicht spezifizierten Reste R bestehen aus Wasserstoff;
- Fig. 5: einen weiteren auf Cyclen basierenden Ansatz gemäß Beispiel 2, wobei die beiden Komplex-bildenden Carboxyldonoren wiederum an sich gegenüberliegenden Ringstickstoffatome gebunden sind. Als Spacer dient wiederum eine 3-Oxapentylkette. Im Gegensatz zu Beispiel 1 werden hier zwei verschiedene Aminosäuren als Targetingvektoren verwendet. Es handelt sich um einen symmetrischen sechzähnigen Chelator mit zwei verschiedenen Aminosäuren L-Tyrosin und L-Serin. L-Tyrosin ist darüber hinaus über eine Aryl-Alkyletherfunktion, das L-Serin über eine Alkyl-etherfunktion an den Spacer gekoppelt;

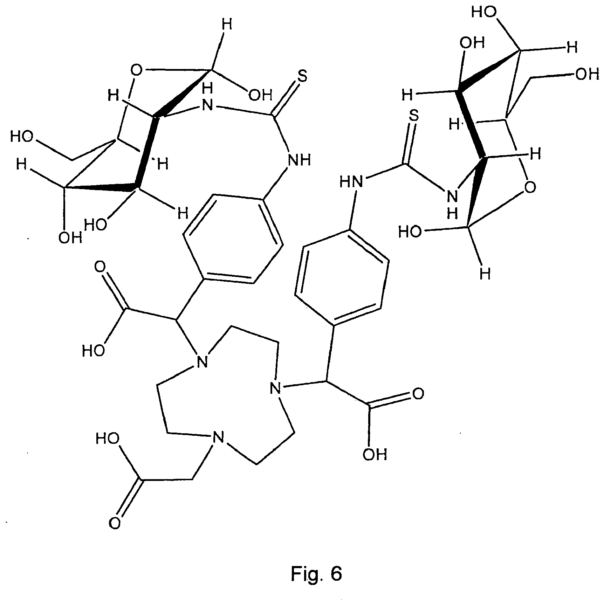

- Fig. 6: einen Ansatz gemäß Beispiel 3, bei dem keine Aminosäuren als TV eingesetzt werden. Auf Basis des 1,4,7-Triazacyclononans, bei dem die 3 Ringstickstoffatome nicht symmetrisch alkyliert sind, dienen hier zwei DesoxyglukoseDerivate als TV. Dieser auf NOTA basierende Chelator weist eine AcetatGruppe und zwei Phenylacetat-Gruppen, Thioharnstofflinker und zwei Desoxyglukose-Derivate als Targetingvektoren auf. Während die Position 1 einen einfachen Donorrest auf Basis der Essigsäure enthält, sind die Substituenten in Position 4 und 7 verzweigt, um die Anbindung eines TV zu ermöglichen. Als Spacer dient in diesem Fall der planare Phenylring, in dessen Position 4 der reaktive Isothiocyanat/ Thioharnstofflinker steht. Als Targetingvektor dienen in diesem Fall die terminal über Spacer und Linker an zueinander benachbarten Stickstoffatomen angebrachten Glukoseeinheiten. Die nicht spezifizierten Reste R bestehen in diesem Fall ebenfalls aus Wasserstoff; und

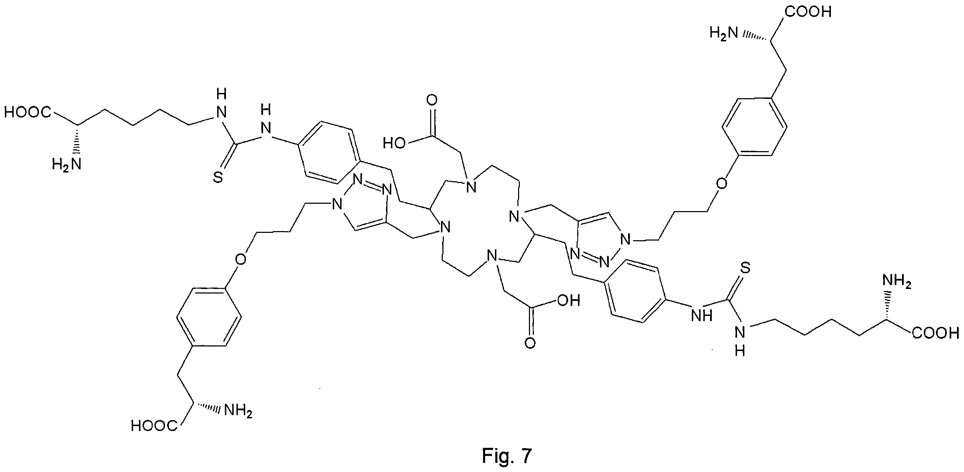

- Fig. 7: eine Kombination zweier orthogonal adressierbarer Linkerfunktionen gemäß Beispiel 4, die die Einführung zweier identischer oder voneinander verschiedener TV ermöglichen. Zwei L-Lysin-TV sind über einen Ethyl-Phenyl-Spacer und eine Isothiocyanat/Thioharnstofffunktion direkt mit dem Rückgrat des makrozyklischen Polyamins verbunden, während zwei weitere L-Tyrosin-TV über einen Propylspacer mit einem 1,2,3-Triazollinker aus einer Huisgen-1,3-dipolaren Cycloaddition an den Chelator gekoppelt wurden. Somit wurden zwei voneinander verschiedene Targetingvektoren jeweils dimerartig mit dem Chelator verknüpft. Die nicht spezifizierten Reste R bestehen in diesem Fall aus Wasserstoff. Es handelt sich um D02A mit zwei L-Tyrosin-TV (verknüpft über eine Huisgen-1,3-dipolare Cycloaddition) und zwei L-Lysin-TV, die über eine backbone-Verzweigung mit Thioharnstofflinkern gekoppelt sind.

Die erfindungsgemäßen Verbindungen unterscheiden sich von bekannten Verbindungen, wie sie z.B. in "Improving Tumor Uptake and Pharmacokinetics of ⁶⁴Cu-Labeled Cyclic RGD Peptide Dimers with Gly₃ and PEG₄ Linkers"; Jiyun Shi^{†}, Young-Seung Kim^{†}, Shizhen Zhai^{†}, Zhaofei Liu^{†}, Xiaoyuan Chen^{‡} and Shuang Liu*^{†}; Publication Date (Web): March 25, 2009 DOI:10.1021/bc800455p, offenbart sind, durch die Verzweigung am Chelator mit zwei unterschiedlichen bioaktiven Molekülen bzw. Targetingvektoren. Erfindungsgemäß werden zwei direkt am Chelator lokalisierte Funktionen bereitgestellt, wobei die beiden Funktionen aufgrund der strukturellen Kombination Spacer-Linker-Targetingvektor unabhängig voneinander ausgebildet sind. Die strukturelle Kombination Spacer-Linker-Targetingvektor vergrößert den Abstand zwischen den beiden Targetingvektoren in beträchtlichem Maße und gestattet es, die Funktionen der beiden Targetingvektoren unabhängig voneinander auszubilden.

Die Funktionalität der erfindungsgemäßen Verbindung zeigt sich in Zellexperimenten anhand ihrer spezifischen Anreicherung.

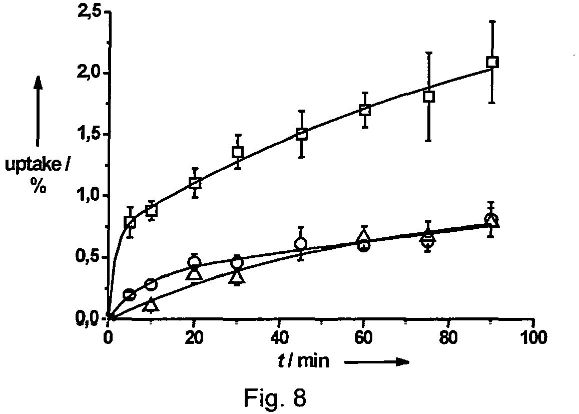

In Fig. 8 ist der zeitliche Verlauf der Aktivitätsanreicherung in F98-Glioblastomzellen wiedergegeben. Man erkennt die Anreicherung von [⁶⁸Ga]Ga-1,4,7,10-tetraazacyclododecan-1,7-diessigsäure-4,10-di-(O-butyl)-L-tyrosin (zugehörige Meßpunkte sind als Quadrate dargestellt), sowie die Anreicherung von [⁶⁸Ga]Ga-DO2A (Kreise) zur Bestimmung der unspezifischen Anreicherung. Im weiteren zeigt Fig. 8 die Resultate eines Blockexperiments (zugehörige Meßpunkte sind als Dreiecke dargestellt), in dem durch Zugabe einer Mischung aus L-Serin, L-Tryptophan und 2-aminobicyclo(2,2,1)heptan-2-carbonsäure (BCH) (jeweils 5 mM) die Anreicherung von [⁶⁸Ga]Ga-1,4,7,10-tetraazacyclododecan-1,7-diessigsäure-4,10-di-(O-butyl)-L-tyrosin auf das Niveau von [⁶⁸Ga]Ga-DO2A verringert werden konnte.

### Ausführungsbeispiele

### Beispiel 1

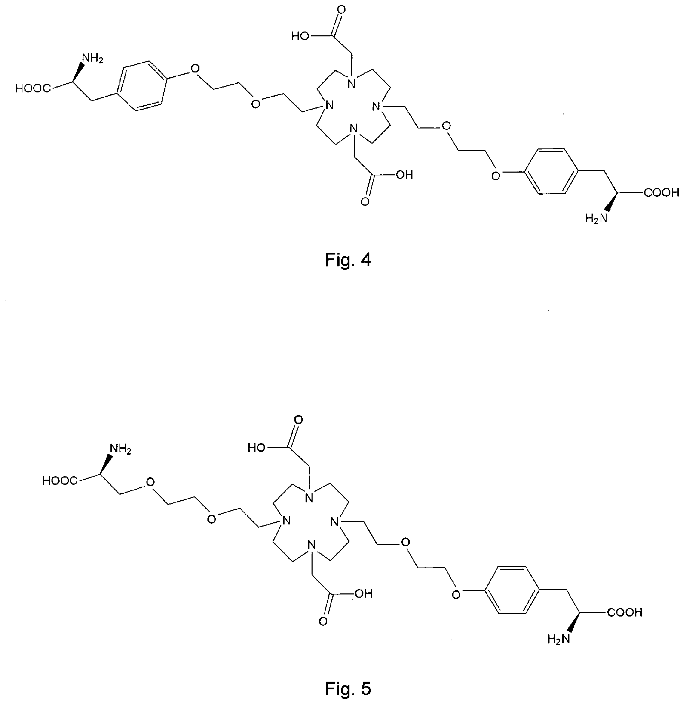

zeigt in Fig. 4 einen auf Cyclen basierenden Ansatz. Die beiden Komplex-bildenden Carboxyldonoren sind an sich gegenüberliegenden Ringstickstoffatome gebunden. Als Spacer dient wiederum eine 3-Oxapentylkette. Zwei gleiche Aminosäuren werden als Targetingvektoren verwendet. Die in Fig. 4 nicht spezifizierten Reste R bestehen aus Wasserstoff.

Die Markierungsvorläufer sind aus kommerziell erhältlichen Chemikalien aufgebaut, die in lehrbuchbekannten (vgl. z. B. K Schwetlick, Organikum, Wiley VCH: Heidelberg (2009); Science of Synthesis, Thieme: Stuttgart, 2002-2009) Routineschritten, z. B. durch nukleophile Substitutionen, miteinander umgesetzt werden. Beispielhaft für die erfindungsgemäßen Verbindungen sind die ¹H-NMR Daten, aufgenommen mit einem Bruker AC 200 FT-NMR, für das in Fig. 4 gezeigte Ausführungsbeispiel 1 angegeben.

¹H NMR (300 MHz, D₂O): δ (in ppm) 7.21 (d, J = 8 Hz, 4H; O-C_{Ar}-C_{Ar}*H*), 6.94 (d, J = 8 Hz, 4H; CH₂-C_{Ar}-C_{Ar}*H*), 4.22 (dd, J = 6 Hz, J = 7 Hz, 2H; *CH-*NH₂), 4.18-4.12 (m, 4H; C_{Ar}O-*CH*₂), 3.95-3.88 (m, 4H; C_{Ar}-O-CH₂-*CH*₂), 3.83-3.78 (m, 4H; N-CH₂-CH₂-O), 3.57-3.50 (m, 4H; N-CH₂-CH₂-O), 3.46 (s, 4H; C*H*₂*-*COOH), 3.50-3.35 (m, 8H; C*H*₂-N-CH₂-COOH), 3.22 (dd, J = 6 Hz, J = 15 Hz, 2H; C_{Ar}-C*H₂*-CH) 3.12 (dd, J = 7 Hz, J = 15 Hz; C_{Ar}-C*H₂*-CH), 3.10-2.91 (m, 8H; C*H₂*-N-CH₂-CH₂-CH₂)

### Beispiel 2

zeigt in Fig. 5 einen auf Cyclen basierenden Ansatz. Die beiden Komplex-bildenden Carboxyldonoren sind wiederum an sich gegenüberliegenden Ringstickstoffatome gebunden. Als Spacer dient wiederum eine 3-Oxapentylkette. Im Gegensatz zu Beispiel 1 werden hier zwei verschiedene Aminosäuren als Targetingvektoren verwendet. L-Tyrosin ist darüber hinaus über eine Aryl-Alkyletherfunktion, das L-Serin über eine Alkyletherfunktion an den Spacer gekoppelt.

Es handelt sich um einen symmetrischen sechzähnigen Chelator mit zwei verschiedenen Aminosäuren L-Tyrosin und L-Serin (s. Fig. 5).

### Beispiel 3

zeigt in Fig. 6 einen alternativen Ansatz bei dem keine Aminosäuren als TV eingesetzt werden. Auf Basis des 1,4,7-Triazacyclononans, bei dem die 3 Ringstickstoffatome nicht symmetrisch alkyliert sind, dienen hier zwei Desoxyglukose-Derivate als TV. Während die Position 1 einen einfachen Donorrest auf Basis der Essigsäure enthält, sind die Substituenten in Position 4 und 7 verzweigt, um die Anbindung eines TV zu ermöglichen. Als Spacer dient in diesem Fall der planare Phenylring, in dessen Position 4 der reaktive Isothiocyanat/Thioharnstofflinker steht. Als Targetingvektor dienen in diesem Fall die terminal über Spacer und Linker an zueinander benachbarten Stickstoffatomen angebrachten Glukoseeinheiten. Die in Fig. 6 nicht spezifizierten Reste R bestehen in diesem Fall ebenfalls aus Wasserstoff.

Dieser auf NOTA basierende Chelator weist eine Acetat-Gruppe und zwei Phenylacetat-Gruppen, Thioharnstofflinker und zwei Desoxyglukose-Derivate als Targetingvektoren auf (s. Fig. 6).

### Beispiel 4

zeigt in Fig. 7 die Kombination zweier orthogonal adressierbarer Linkerfunktionen, die die Einführung zweier identischer oder voneinander verschiedener TV ermöglichen. Zwei L-Lysin-TV sind über einen Ethyl-Phenyl-Spacer und eine Isothiocyanat/Thioharnstofffunktion direkt mit dem Rückgrat des makrozyklischen Polyamins verbunden, während zwei weitere L-Tyrosin-TV über einen Propylspacer mit einem 1,2,3-Triazollinker aus einer Huisgen-1,3-dipolaren Cycloaddition an den Chelatorgekoppelt wurden. Somit wurden zwei voneinander verschiedene Targetingvektoren jeweils dimerartig mit dem Chelator verknüpft. Die in Fig. 7 nicht spezifizierten Reste R bestehen in diesem Fall aus Wasserstoff. Es handelt sich um D02A mit zwei L-Tyrosin-TV (verknüpft über eine Huisgen-1,3-dipolare Cycloaddition) und zwei L-Lysin-TV, die über eine backbone-Verzweigung mit Thioharnstofflinkern gekoppelt sind (s. Fig. 7).

## Patentansprüche

1. Verbindung aus einem metallischen Radionuklid und einem Markierungsvorläufer mit einem bifunktionellen, makrozyklischen Chelator gewählt aus der Gruppe, umfassend 9- bis 16-gliedrige Polyazamakrozyklen und makrozyklisches Polyamin, das an allen Stickstoffatomen substituiert ist und worin das zentrale metallische Radionuklid mit den benachbarten Stickstoffatomen 5-Ring-Chelate oder 6-Ring-Chelate bildet, **dadurch gekennzeichnet, dass** der Markierungsvorläufer aus dem Chelator und zumindest zwei, über Kopplungseinheiten mit dem Chelator vebundenen und voneinander verschiedenen Targetingvektoren aufgebaut ist, wobei die Kopplungseinheiten aus einem Spacer bestehen, der Spacer eine lineare, verzweigte oder heterosubstituierte Alkyl-, Alkylen-, Alkyliden-, Aryl-, Polyether- oder Peptidkette ist, die kovalent unter Beibehaltung ihrer Aminofunktionalität gebunden ist; und
die Targetingvektoren aus proteinogenen oder künstlichen Aminosäuren, die jeweils eine α-Amino-Carbonsäure-Gruppe aufweisen, aufgebaut sind oder gewählt sind aus Bisphosphonaten, Zucker, Purin- und Pyrimidinbasen, Nukleotiden, Nukleosiden und deren Derivaten, Antikörpern, Antikörperfragmenten, DNA-Komponenten, Hypoxie-Tracern, Zellmembran-Komponenten, Glutamin, extra- oder interzelluläre Enzym- oder Rezeptorliganden.

2. Verbindung nach Anspruch 1, **dadurch gekennzeichnet, dass** an den Chelator zwei oder mehrere Kombinationen aus Kopplungseinheit und Targetingvektor gebunden ist.

3. Verbindung nach Anspruch 1, **dadurch gekennzeichnet, dass** jede Kombination aus Kopplungseinheit und Targetingvektor an den Chelator jeweils über verschiedene Ringstickstoff-Atome und/oder verschiedene Kohlenstoffatome des Chelators gebunden ist.

4. Verbindung nach Anspruch 1, **dadurch gekennzeichnet, dass** der Chelator komplexbildende Donoren aus der Gruppe CNHOH, CSOH, COOH, CH₂OH, CH₂SH, PO(OH)₂, CH₂SO₃H enthält.

5. Verbindung nach einem der Ansprüche 1 und 4, **dadurch gekennzeichnet, dass** ein Chelator verwendet wird, der aus Polyaminopolycarbonsäuren und gegebenenfalls komplexbildenden Donoren aus der Gruppe CNHOH, CSOH, COOH, CH₂OH, CH₂SH, PO(OH)₂, CH₂SO₃H aufgebaut ist.

6. Verbindung nach einem der Ansprüche 1,4 und 5, **dadurch gekennzeichnet, dass** ein Chelator verwendet wird, der aus Polyaminopolycarbonsäuren aufgebaut ist, die zusätzlich oder anstelle einer oder mehrerer Carbonsäureeinheiten auch SH-Gruppen enthalten.

7. Verbindung nach einem der Ansprüche 1 bis 6, **dadurch gekennzeichnet, dass** die an der Ringstruktur des makrozyklischen Polyamins substituierten Reste R aus der Gruppe umfassend Wasserstoff, Alkyl, Aryl, Heteroaryl, Alkenyl, Alkinyl, Kombination aus Kopplungseinheit/Targetingvektor ausgewählt sind.

8. Verbindung nach Anspruch 7, **dadurch gekennzeichnet, dass** alle Reste R identisch oder verschieden voneinander sind.

9. Verbindung nach einem der Ansprüche 1 bis 8, **dadurch gekennzeichnet, dass** der Chelator mit zwei-, drei- oder vierwertigen metallischen Radionukliden markiert ist.

10. Verbindung nach Anspruch 1, **dadurch gekennzeichnet, dass** die verschiedenen Targetingvektoren das gleiche oder verschiedene biologische Target(s) adressieren.

## Claims

1. Compound composed of a metallic radionuclide and a marker precursor having a bifunctional, macrocyclic chelator selected from the group comprising 9- to 16-membered polyazamacrocycles and macrocyclic polyamine substituted on all nitrogen atoms and wherein the central metallic radionuclide forms 5-ring chelates or 6-ring chelates with the adjacent nitrogen atoms, **characterized in that** the marker precursor is composed of the chelator and at least two targeting vectors different from each other and linked to the chelator via coupling units, wherein the coupling units consist of a spacer, said spacer being a linear, branched or heterosubstituted alkyl, alkylene, alkylidene, aryl, polyether or peptide chain which is covalently bonded while maintaining its amino functionality; the targeting vectors are composed of proteinogenic or artificial amino acids each having an α-aminocarboxylic acid group or are selected from bisphosphonates, sugars, purine and pyrimidine bases, nucleotides, nucleosides and derivatives thereof, antibodies, antibody fragments, DNA components, hypoxia tracers, cell membrane components, glutamine, extracellular or intracellular enzyme or receptor ligands.

2. Compound according to Claim 1, **characterized in that** two or more combinations of coupling unit and targeting vector is bonded to the chelator.

3. Compound according to Claim 1, **characterized in that** each combination of coupling unit and targeting vector is bonded to the chelator via different ring nitrogen atoms and/or different carbon atoms of the chelator in each case.

4. Compound according to Claim 1, **characterized in that** the chelator comprises complexing donors from the group of CNHOH, CSOH, COOH, CH₂OH, CH₂SH, PO(OH)₂, CH₂SO₃H.

5. Compound according to either of Claims 1 and 4, **characterized in that** a chelator is used which is composed of polaminopolycarboxylic acids and optionally complexing donors from the group of CNHOH, CSOH, COOH, CH₂OH, CH₂SH, PO(OH)₂, CH₂SO₃H.

6. Compound according to any of Claims 1, 4 and 5, **characterized in that** a chelator is used which is composed of polyaminopolycarboxylic acids also comprising SH groups in addition to or instead of one or more carboxylic acid units.

7. Compound according to any of Claims 1 to 6, **characterized in that** the radicals R substituted on the ring structure of the macrocyclic polyamine are selected from the group comprising hydrogen, alkyl, aryl, heteroaryl, alkenyl, alkynyl, combination of coupling unit/targeting vector.

8. Compound according to Claim 7, **characterized in that** all radicals R are identical or different from one another.

9. Compound according to any of Claims 1 to 8, **characterized in that** the chelator is labelled with divalent, trivalent or tetravalent metallic radionuclides.

10. Compound according to Claim 1, **characterized in that** the different targeting vectors address the same or different biological target(s).

## Revendications

1. Composé à partir d'un radionucléide métallique et d'un précurseur de marquage comprenant un chélateur bifonctionnel, macrocyclique choisi parmi le groupe comprenant des polyaza-macrocycles à 9 à 16 membres et une polyamine macrocyclique, qui est substitué au niveau de tous les atomes d'azote, et dans lequel le radionucléide métallique central forme, avec les atomes d'azote adjacents, des chélates à 5 cycles ou des chélates à 6 cycles, **caractérisé en ce que** le précurseur de marquage présente une structure formée à partir du chélateur et au moins de deux vecteurs de ciblage reliés au chélateur par l'intermédiaire d'unités de couplage et différents les uns des autres, dans lequel les unités de couplage sont constituées d'un espaceur, l'espaceur est une chaîne alkyle, une chaîne alkylène, une chaîne alkylidène, une chaîne aryle, une chaîne polyether ou une chaîne peptidique linéaire, ramifiée ou hétérosubstituée, qui est liée de manière covalente en conservant sa fonctionnalité amino ; et
**en ce que** les vecteurs de ciblage présentent une structure formée à partir d'acides aminés protéinogéniques ou synthétiques, qui présentent respectivement un groupe α-amino-acide carboxylique, ou sont choisis parmi des biphosphonates, le sucre, des bases puriques et des bases pyrimidiques, des nucléotides, des nucléosides et leurs dérivés, des anticorps, des fragments d'anticorps, des composants d'ADN, des traceurs d'hypoxie, des composants de membrane cellulaire, la glutamine, des ligands d'enzyme ou de récepteur extra- ou intracellulaires.

2. Composé selon la revendication 1, **caractérisé en ce que** deux ou plusieurs combinaisons composées d'une unité de couplage et d'un vecteur de ciblage sont liées au chélateur.

3. Composé selon la revendication 1, **caractérisé en ce que** chaque combinaison composée d'une unité de couplage et d'un vecteur de ciblage est liée au chélateur respectivement par l'intermédiaire de différents atomes d'azote cyclique et/ou de différents atomes de carbone du chélateur.

4. Composé selon la revendication 1, **caractérisé en ce que** le chélateur contient des donneurs complexants issus du groupe CNHOH, CSOH, COOH, CH₂OH, CH₂SH, PO(OH)₂, CH₂SO₃H.

5. Composé selon l'une quelconque des revendications 1 et 4, **caractérisé en ce qu'**un chélateur est utilisé, lequel présente une structure formée à partir d'acides polyamino-polycarboxyliques et éventuellement de donateurs complexants issus du groupe CNHOH, CSOH, COOH, CH₂OH, CH₂SH, PO(OH)₂, CH₂SO₃H.

6. Composé selon l'une quelconque des revendications 1, 4 et 5, **caractérisé en ce qu'**un chélateur est utilisé, lequel présente une structure formée à partir d'acides polyamino-polycarboxyliques, qui contiennent, en supplément ou en lieu et place d'un ou de plusieurs motifs d'acide carboxylique, également des groupes SH.

7. Composé selon l'une quelconque des revendications 1 à 6, **caractérisé en ce que** les radicaux R substitués au niveau de la structure cyclique de la polyamine macrocyclique sont choisis parmi le groupe comprenant l'hydrogène, l'alkyle, l'aryle, l'hétéroaryle, l'alcényle, l'alkynyle, la combinaison composée d'une unité de couplage/d'un vecteur de ciblage.

8. Composé selon la revendication 7, **caractérisé en ce que** tous les radicaux R sont identiques ou différents les uns des autres.

9. Composé selon l'une quelconque des revendications 1 à 8, **caractérisé en ce que** le chélateur est marqué par des radionucléides métalliques bi-, tri- ou tétravalents.

10. Composé selon la revendication 1, **caractérisé en ce que** les différents vecteurs de ciblage visent la même cible biologique ou des cibles biologiques différentes.
